# EUROPEAN PATENT APPLICATION

(11) **EP 4 806 404 A1**
(43) Date of publication of application: **16.09.2026**
(21) Application number: 24888756.4
(22) Date of filing: 07.11.2024
(51) Int. Cl.: A61F 9/007

(54) **CELL TRANSPLANTATION INSTRUMENT**

(30) Priority: 10.11.2023 JP 2023191981
(71) Applicant: PUBLIC UNIVERSITY CORPORATION NAGOYA CITY UNIVERSITY, Nagoya-shi, Aichi 467-8601 (JP)
(72) Inventor: YASUKAWA, Tsutomu, Nagoya-shi, Aichi 467-8601 (JP)
(74) Representative: Kuhnen & Wacker Patent- und Rechtsanwaltsbüro PartG mbB
(86) International application number: PCT/JP2024/039528
(87) International publication number: WO 2025/100463

(57) **Abstract**

Provided is a technique capable of improving the operability in subretinal cell transplantation. A cell transplantation instrument to the subretinal space includes: a loading portion having a tubular shape and including a first through-hole formed along a longitudinal direction, the first through-hole being loaded with transplanted cells; and a grip portion continuous with a proximal end of the loading portion and having a shape to be gripped with forceps, wherein a flow passage communicating with the first through-hole is formed inside the grip portion.

## Description

### [Technical Field]

The present disclosure relates to a cell transplantation instrument. The present application is based on Japanese Patent Application No. 2023-191981 filed on November 10, 2023, the contents of which are incorporated herein by reference.

### [Background Art]

For regenerative medicine for retinal vitreous diseases such as age-related macular degeneration, clinical studies for transplanting a cell sheet of iPS cell-derived retinal pigment epithelial cells or the like to the subretinal space have been conducted. Since a large incision is required for the transplantation of the cell sheet, it is difficult to insert an instrument and the risk of subretinal hemorrhage is high in an extraocular approach in which the cell sheet is transplanted to the subretinal space after incising the sclera/choroid. For this reason, it is necessary to transplant the cell sheet by an intraocular approach in which the cell sheet is transplanted to the subretinal space through a vitreous cavity/retina incision site through the sclera/pars plana (or the corneosclera). For example, PTL 1 discloses a device used by being inserted into the vitreous cavity through a sclera/pars plana incision port.

### [Citation List]

### [Patent Literature]

[PTL 1] WO 2010/085693 A

### [Summary of Invention]

### [Technical Problem]

The device described in PTL 1 has room for improvement in terms of operability. For this reason, there has been a demand for a technique capable of improving the operability in subretinal cell transplantation.

### [Solution to Problem]

The present invention can be implemented in the following modes.
(1) According to one mode of the present disclosure, a cell transplantation instrument to the subretinal space is provided. The cell transplantation instrument of this mode is a cell transplantation instrument to a subretinal space, the cell transplantation instrument including: a loading portion having a tubular shape and including a first through-hole formed along a longitudinal direction, the first through-hole being loaded with transplanted cells; and a grip portion continuous with a proximal end of the loading portion and having a shape to be gripped with forceps, wherein a flow passage communicating with the first through-hole is formed inside the grip portion. According to the cell transplantation instrument of this mode, since the grip portion can be gripped with the forceps and the loading portion can be guided to a desired position, the operability in subretinal cell transplantation can be improved.
(2) In the cell transplantation instrument according to the above (1), the shape may be formed by a protrusion. According to the cell transplantation instrument of this mode, since the protrusion can be gripped with the forceps and the loading portion can be guided to a desired position, the operability in subretinal cell transplantation can be improved.
(3) The cell transplantation instrument according to the above (1) or (2) may further include a hollow tube continuous with a proximal end of the grip portion and including a second through-hole formed along a longitudinal direction of the hollow tube, wherein the hollow tube may have flexibility, and the second through-hole may communicate with the flow passage. According to the cell transplantation instrument of this mode, since the hollow tube in which the second through-hole communicating with the flow passage is formed is further provided, it is possible to easily inject a liquid into the cell transplantation instrument and easily transplant the cells loaded in the loading portion.
(4) The cell transplantation instrument according to the above (1) or (2) may further include a hollow needle continuous with a proximal end of the grip portion and including a third through-hole formed along a longitudinal direction of the hollow needle, wherein the hollow needle may have rigidity, and the third through-hole may communicate with the flow passage. According to the cell transplantation instrument of this mode, since the hollow needle in which the third through-hole communicating with the flow passage is formed is further provided, it is possible to easily inject a liquid into the cell transplantation instrument and easily transplant the cells loaded in the loading portion.
(5) The cell transplantation instrument according to the above (1) or (2) may further include a thread or a wire continuous with a proximal end of the grip portion. According to the cell transplantation instrument of this mode, since the thread or the wire continuous with the proximal end of the grip portion is further provided, the cell transplantation instrument can be easily removed.
(6) In the cell transplantation instrument according to any one of the above (1) to (5), the grip portion may further include a connection portion, and the connection portion may be connected to a hollow conduit that is introduced from an outside through a scleral wound, to bring the flow passage of the grip portion and an inside of the hollow conduit into communication with each other. According to the cell transplantation instrument of this mode, since the connection portion that brings the flow passage of the grip portion and the inside of the hollow conduit into communication with each other is provided, it is possible to inject the liquid into the cell transplantation instrument through the hollow conduit and transplant the cells loaded in the loading portion.
(7) In the cell transplantation instrument according to any one of the above (1) to (6), a maximum dimension of the grip portion in a cross section perpendicular to the longitudinal direction may be substantially equal to or smaller than a maximum dimension of the loading portion in the cross section. According to the cell transplantation instrument of this mode, since the maximum dimension of the grip portion in the cross section is substantially equal to or smaller than the maximum dimension of the loading portion, it is possible to reduce or prevent interference of the grip portion at the time of inserting or removing the cell transplantation instrument into or from a scleral wound, for example.
(8) In the cell transplantation instrument according to any one of the above (1) to (7), a distal end of the loading portion may have an obliquely-cut shape. According to the cell transplantation instrument of this mode, since the distal end of the loading portion has an obliquely-cut shape, the loading portion can be easily inserted into the scleral wound or an incision of the retina.
(9) In the cell transplantation instrument according to the above (8), when the loading portion is placed with a cut surface of the distal end facing upward, the shape may also be provided to face upward. According to the cell transplantation instrument of this mode, when the loading portion is placed with the cut surface of the distal end facing upward, the shape to be gripped with the forceps in the grip portion is also provided to face upward, so that the operability at the time of inserting the distal end of the loading portion into the incision of the retina can be improved.

Note that the present disclosure can be implemented in various modes, and can be implemented, for example, in the modes of a method of manufacturing the cell transplantation instrument to the subretinal space, a method of using the cell transplantation instrument to the subretinal space, a method of assisting treatment using the cell transplantation instrument to the subretinal space, a treatment method using the cell transplantation instrument to the subretinal space, and the like.

### [Brief Description of Drawings]

[Fig. 1] An explanatory view schematically illustrating a schematic configuration of a cell transplantation instrument.
[Fig. 2] An explanatory view illustrating an example of a use state of the cell transplantation instrument.
[Fig. 3] An explanatory view illustrating an example of a use state of a cell transplantation instrument of a comparative example.
[Fig. 4] An explanatory view schematically illustrating a schematic configuration of a cell transplantation instrument according to a second embodiment.
[Fig. 5] An explanatory view illustrating an example of a use state of the cell transplantation instrument according to the second embodiment.
[Fig. 6] An explanatory view schematically illustrating a schematic configuration of a cell transplantation instrument according to a third embodiment.
[Fig. 7] An explanatory view illustrating an example of a use state of the cell transplantation instrument according to the third embodiment.

### [Description of Embodiments]

### A. First Embodiment

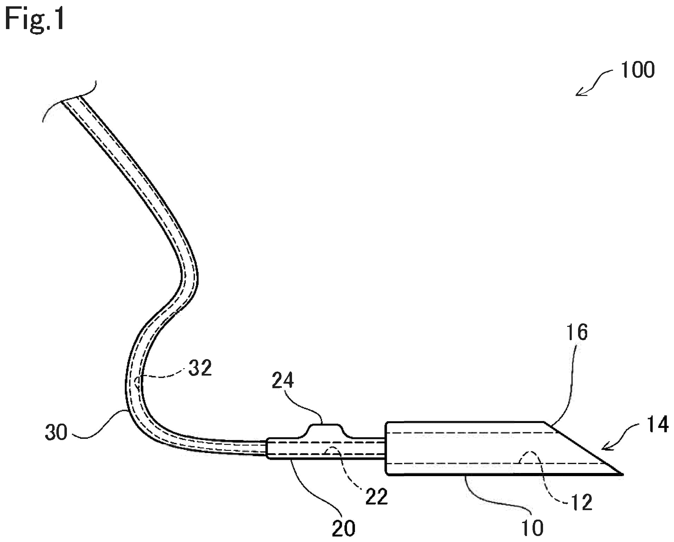

Fig. 1 is an explanatory view schematically illustrating a schematic configuration of a cell transplantation instrument 100 as one embodiment of the present disclosure. The cell transplantation instrument 100 of the present disclosure is inserted into the vitreous cavity through a scleral wound such as a sclera/pars plana incision port, and is used for transplanting cells to the subretinal space. The transplanted cells are not particularly limited, and examples thereof include pluripotent stem cells such as induced pluripotent stem cells (iPS cells) and embryonic stem cells (ES cells), a cell sheet and a cell cluster prepared using somatic cells collected/cultured from a living body, and a cell sheet (amniotic membrane or the like) collected from a living body. The subretinal cell transplantation is performed, for example, in regenerative medicine such as cell replacement therapy for retinal pigment epithelium dysfunction or the like. The retinal pigment epithelium dysfunction is not particularly limited, and examples thereof include age-related macular degeneration, retinitis pigmentosa, angioid streaks, chorioretinal atrophy associated with pathologic myopia, Best disease, and bull's-eye maculopathy.

As illustrated in Fig. 1, the cell transplantation instrument 100 of the present embodiment includes a loading portion 10, a grip portion 20, and a hollow tube 30. The loading portion 10, the grip portion 20, and the hollow tube 30 are arranged in this order from the distal end side to the proximal end side in an insertion direction of the cell transplantation instrument 100. Since each component in Fig. 1 is schematically illustrated, the size of each component, member, or the like is different from the actual size.

The loading portion 10 has a substantially tubular outer shape in which a first through-hole 12 is formed along a longitudinal direction of the loading portion 10. The first through-hole 12 is loaded with the transplanted cells. In the present embodiment, a distal end 14 of the loading portion 10 has an obliquely-cut shape. In other words, the distal end 14 of the loading portion 10 has a shape cut along a cut surface 16 intersecting the longitudinal direction and a radial direction. Since the distal end 14 of the loading portion 10 has an obliquely-cut shape, the loading portion 10 can be easily inserted into the scleral wound, and the distal end 14 of the loading portion 10 can be easily inserted into an incision of the retina. The loading portion 10 is formed to have an outer diameter of, for example, about 1.3 mm to 2.0 mm. The loading portion 10 in the present embodiment is formed of a transparent or translucent member, to allow the cells loaded in the first through-hole 12 to be seen through. The loading portion 10 is formed of, for example, a resin material such as Teflon (registered trademark), acrylic, or silicon.

The grip portion 20 is continuous with a proximal end of the loading portion 10. A flow passage 22 communicating with the first through-hole 12 of the loading portion 10 is formed inside the grip portion 20. The grip portion 20 of the present embodiment has a substantially tubular outer shape. The flow passage 22 of the present embodiment is formed to penetrate in a longitudinal direction. The grip portion 20 includes a protrusion 24 to be gripped with forceps described later. In the present embodiment, the protrusion 24 is formed to protrude radially outward. In addition, in the present embodiment, the protrusion 24 has a thin plate-like outer shape extending along the longitudinal direction of the grip portion 20. In the present embodiment, when the loading portion 10 is placed with the cut surface 16 of the distal end 14 facing upward, the protrusion 24 is also provided to face upward. Therefore, an opening direction of the first through-hole 12 at the distal end 14 and a protruding direction of the protrusion 24 substantially coincide with each other in a circumferential direction of the loading portion 10. Such a configuration makes it possible to improve the operability at the time of inserting the distal end 14 of the loading portion 10 into the incision of the retina. The grip portion 20 is formed of, for example, a resin material such as Teflon (registered trademark), acrylic, or silicon.

In the present embodiment, a maximum dimension of the grip portion 20 in a cross section perpendicular to the longitudinal direction is substantially equal to or smaller than a maximum dimension of the loading portion 10 in the cross section perpendicular to the longitudinal direction. In the present disclosure, "substantially equal" in dimension means that a difference in dimension is within 10%. The maximum dimension of the grip portion 20 in the cross section perpendicular to the longitudinal direction is preferably smaller than the maximum dimension of the loading portion 10 in the cross section perpendicular to the longitudinal direction. In the present embodiment, the cross section perpendicular to the longitudinal direction corresponds to a cross section along the radial direction. In addition, the maximum dimension of the grip portion 20 in the cross section perpendicular to the longitudinal direction corresponds to the dimension of the grip portion 20 including the protrusion 24. The above dimensional relationship makes it possible to reduce or prevent interference of the protrusion 24 formed on the grip portion 20 at the time of inserting or removing the cell transplantation instrument 100 into or from the sclera/pars plana incision port, for example.

The hollow tube 30 is provided continuously with a proximal end of the grip portion 20, and a second through-hole 32 is formed along a longitudinal direction of the hollow tube 30. The hollow tube 30 has flexibility. A proximal end of the hollow tube 30 is exposed to the outside of the scleral wound. The second through-hole 32 formed in the hollow tube 30 communicates with the flow passage 22 formed in the grip portion 20. In the present embodiment, the hollow tube 30 is formed of, for example, a resin material such as Teflon (registered trademark), acrylic, or silicon.

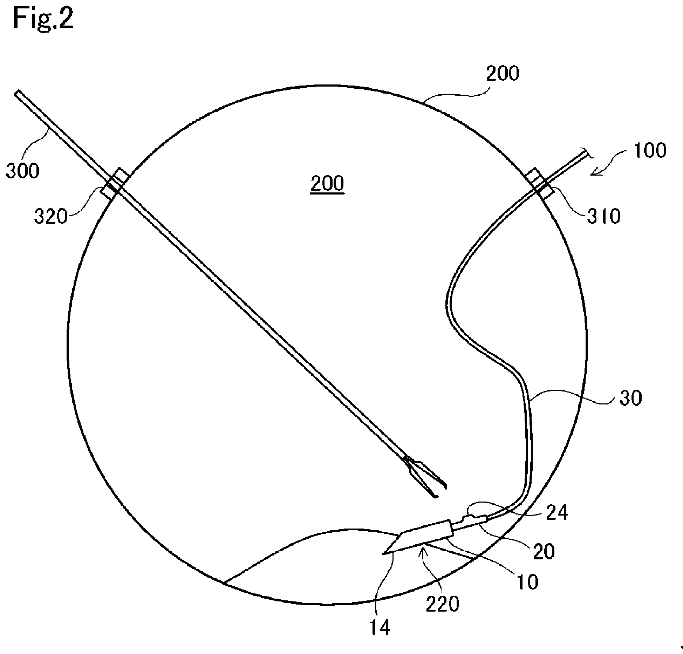

Fig. 2 is an explanatory view illustrating an example of a use state of the cell transplantation instrument 100. Fig. 2 schematically illustrates an eyeball 200 and forceps 300 together with the cell transplantation instrument 100. In Fig. 2, the upper side of the drawing illustrates the anterior segment side (lens side), and the lower side of the drawing illustrates the posterior segment side (optic nerve side). In the example illustrated in Fig. 2, a first sclera/pars plana incision port 310 and a second sclera/pars plana incision port 320 are formed in the eyeball 200 as the scleral wounds.

The first through-hole 12 formed in the loading portion 10 of the cell transplantation instrument 100 is loaded with the cells such as a cell sheet (not illustrated) in advance. In addition, an automatic or manual syringe, pump, or the like (not illustrated) for injecting a liquid through the second through-hole 32 is connected to the proximal end of the hollow tube 30 of the cell transplantation instrument 100.

The cell transplantation instrument 100 is inserted into a vitreous cavity 210 of the eyeball 200 through the first sclera/pars plana incision port 310 with the side on which the loading portion 10 is disposed as the distal end side. In addition, the forceps 300 for gripping the grip portion 20 of the cell transplantation instrument 100 are inserted into the vitreous cavity 210 through the second sclera/pars plana incision port 320. As described above, in the cell transplantation instrument 100 of the present embodiment, the maximum dimension of the grip portion 20 in the cross section perpendicular to the longitudinal direction is substantially equal to or smaller than the maximum dimension of the loading portion 10, so that the interference of the grip portion 20 at the time of insertion is reduced or prevented.

A user of the cell transplantation instrument 100 can freely move the position or angle of the loading portion 10 by gripping the protrusion 24 formed on the grip portion 20 with the forceps 300. In addition, since the hollow tube 30 has flexibility, restriction of the movement of the loading portion 10 is reduced or prevented. The user of the cell transplantation instrument 100 can easily guide the loading portion 10 to an incision site 220 of the retina, and can easily insert the distal end 14 of the loading portion 10 into the incision site 220 of the retina. In a state where the distal end 14 of the loading portion 10 reaches a desired transplantation site in the subretinal space, the cells loaded in the loading portion 10 are transplanted by a water flow. More specifically, a liquid is injected through the second through-hole 32 formed in the hollow tube 30, the flow passage 22 formed in the grip portion 20, and the first through-hole 12 formed in the loading portion 10, whereby the cells in the loading portion 10 are transplanted by being carried on the water flow of the liquid. Thereafter, the cell transplantation instrument 100 is removed through the first sclera/pars plana incision port 310, and the forceps 300 are removed through the second sclera/pars plana incision port 320.

According to the cell transplantation instrument 100 of the present embodiment described above, since the loading portion 10 loaded with the transplanted cells and the grip portion 20 continuous with the proximal end of the loading portion 10 are provided, it is possible to grip the protrusion 24 and guide the loading portion 10 to a desired position. As a result, the operability in subretinal cell transplantation can be improved. As a result of improving the operability in subretinal cell transplantation, a burden on the scleral wound can be reduced, so that dehiscence of the scleral wound can be reduced or prevented. As a result, leakage of intraocular fluid and a decrease in intraocular pressure can be reduced or prevented, so that a decrease in safety can be reduced or prevented. In addition, occurrence of a turbulent flow due to intraocular perfusate can be reduced or prevented, which makes it possible to reduce or prevent a loss of the cell sheet in the eye.

In addition, since the flow passage 22 formed inside the grip portion 20 communicates with the first through-hole 12 of the loading portion 10, the transplanted cells loaded in the first through-hole 12 can be transplanted by being carried on the water flow of the liquid injected through the flow passage 22. Furthermore, since the hollow tube 30 is continuous with the proximal end of the grip portion 20, the liquid can be injected into the flow passage through the hollow tube 30, and the cell transplantation instrument 100 can be easily removed from the eyeball 200 after cell transplantation or the like. Therefore, the hollow tube 30 can serve as both the member for injecting the liquid into the flow passage 22 and the member for removing the cell transplantation instrument 100, and it is possible to reduce or prevent complication of the structure of the cell transplantation instrument 100. In addition, since the hollow tube 30 has flexibility, interference by the hollow tube 30 can be reduced or prevented, and damage in the eyeball 200 can be reduced or prevented.

### B. Comparative Example

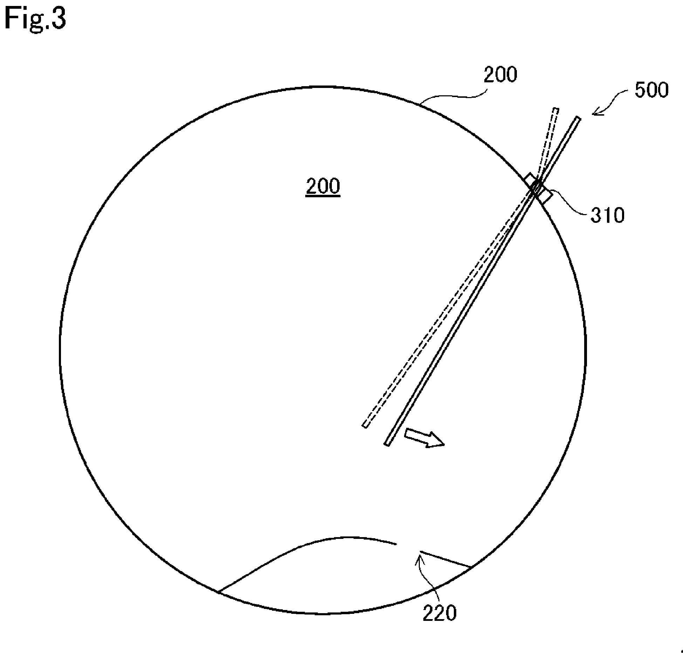

Fig. 3 is an explanatory view illustrating an example of a use state of a cell transplantation instrument 500 of a comparative example. The cell transplantation instrument 500 of the comparative example is inserted into the vitreous cavity 210 of the eyeball 200 through the first sclera/pars plana incision port 310. In the cell transplantation instrument 500 of the comparative example, the grip portion is omitted. In the cell transplantation instrument 500 of the comparative example, a proximal end portion exposed to the outside of the scleral wound is moved to insert a distal end portion into the incision site 220 of the retina. For this reason, the cell transplantation instrument 500 of the comparative example is restricted at two points of the first sclera/pars plana incision port 310 and the incision site 220 of the retina. Thus, the operability is deteriorated. For example, in the cell transplantation instrument 500 having a linear shape formed by a rigid hollow needle or the like, it may be difficult to cause the distal end portion to reach the incision site 220. In addition, for example, in the cell transplantation instrument 500 formed by a flexible hollow tube or the like, it is difficult to move the distal end portion to a desired position with the first sclera/pars plana incision port 310 serving as a fulcrum. More specifically, in a case where it is desired to move the distal end portion in a direction indicated by a white arrow in Fig. 3, the cell transplantation instrument 500 bends in the opposite direction as indicated by a broken line in Fig. 3. Thus, the operability is poor. In addition, particularly in a highly myopic eye, a distance between the two points of the first sclera/pars plana incision port 310 and the incision site 220 of the retina is long. Thus, the operability is further deteriorated.

On the other hand, according to the cell transplantation instrument 100 of the present embodiment, since the protrusion 24 of the grip portion 20 is gripped with the forceps 300, the distal end 14 of the loading portion 10 for transplanting cells can be moved and inserted into the subretinal space without being affected by the position, angle, and the like of the scleral wound, and thus, the operability can be improved.

### C. Second Embodiment

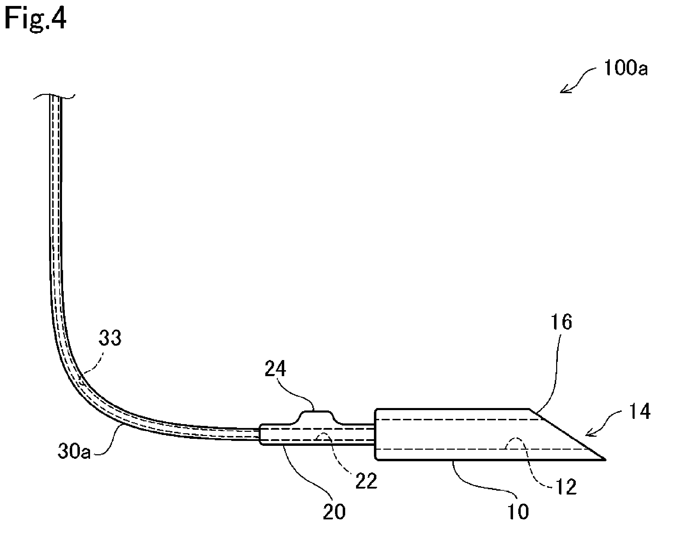

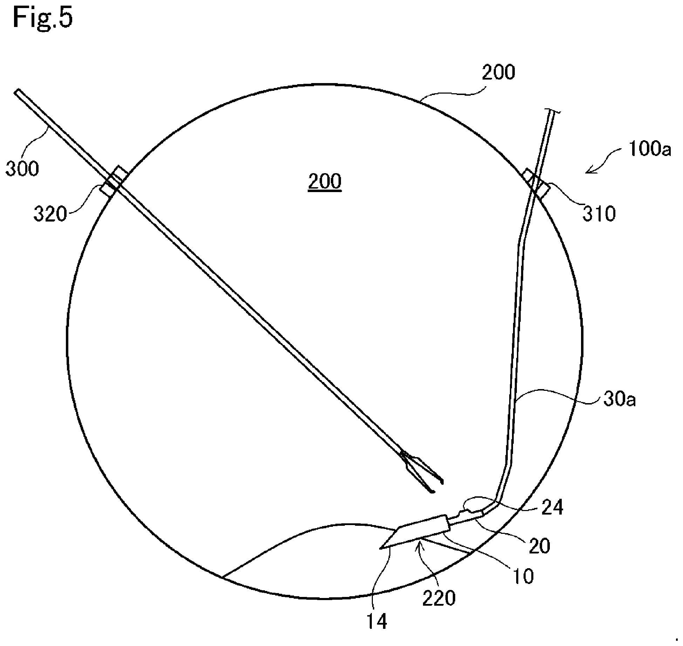

Fig. 4 is an explanatory view schematically illustrating a schematic configuration of a cell transplantation instrument 100a according to a second embodiment. Fig. 5 is an explanatory view illustrating an example of a use state of the cell transplantation instrument 100a according to the second embodiment. The cell transplantation instrument 100a of the second embodiment is different from the cell transplantation instrument 100 of the first embodiment in that a hollow needle 30a is provided instead of the hollow tube 30. Since the other components are similar to those of the cell transplantation instrument 100 of the first embodiment, the same reference numerals are assigned to the same components, and a detailed description thereof will be omitted.

The hollow needle 30a is provided continuously with the proximal end of the grip portion 20, and a third through-hole 33 is formed along a longitudinal direction of the hollow needle 30a. The hollow needle 30a has rigidity. A proximal end of the hollow needle 30a is exposed to the outside of the scleral wound. The third through-hole 33 formed in the hollow needle 30a communicates with the flow passage 22 formed in the grip portion 20. The hollow needle 30a is formed of, for example, a metal material such as stainless steel. The hollow needle 30a is deformed by applying a force from the outside. Therefore, as illustrated in Fig. 5, the cell transplantation instrument 100a may be inserted into the vitreous cavity 210 in a state where the hollow needle 30a is appropriately bent.

According to the cell transplantation instrument 100a of the second embodiment described above, the operability in subretinal cell transplantation can be improved similarly to the cell transplantation instrument 100 of the first embodiment. In addition, since the hollow needle 30a continuous with the proximal end of the grip portion 20 is provided, the diameter of the member continuous with the proximal end of the grip portion 20 can be further reduced. As a result, leakage of intraocular fluid and a decrease in intraocular pressure can be further reduced or prevented, so that a decrease in safety can be further reduced or prevented.

### D. Third Embodiment

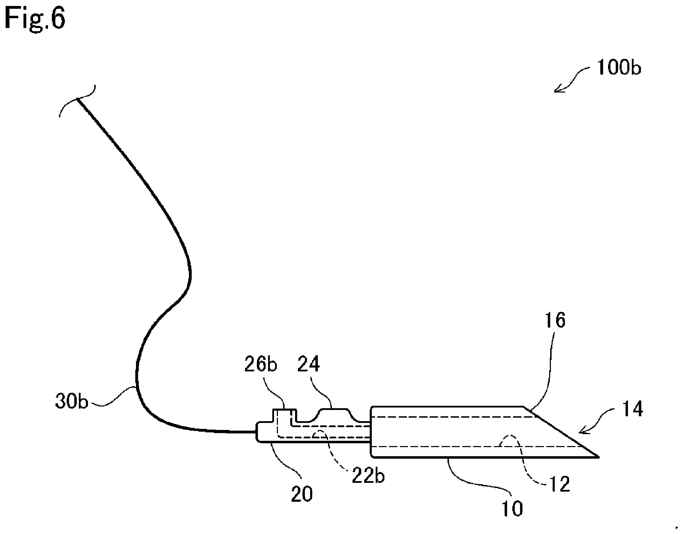

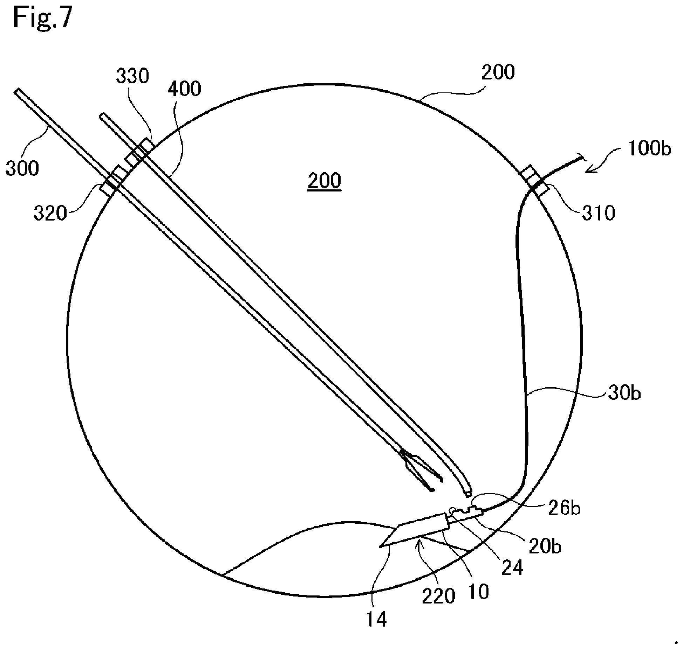

Fig. 6 is an explanatory view schematically illustrating a schematic configuration of a cell transplantation instrument 100b according to a third embodiment. Fig. 7 is an explanatory view illustrating an example of a use state of the cell transplantation instrument 100b according to the third embodiment. The cell transplantation instrument 100b of the third embodiment is different from the cell transplantation instrument 100 of the first embodiment in that a small-diameter member 30b formed by a thread or a wire is provided instead of the hollow tube 30 and that a grip portion 20b is provided instead of the grip portion 20. Since the other components are similar to those of the cell transplantation instrument 100 of the first embodiment, the same reference numerals are assigned to the same components, and a detailed description thereof will be omitted.

The small-diameter member 30b is continuous with a proximal end of the grip portion 20b. For example, the small-diameter member 30b may be firmly tied to the proximal end of the grip portion 20b. Unlike the hollow tube 30 and the hollow needle 30a of the first and second embodiments, no through-hole is formed inside the small-diameter member 30b. The small-diameter member 30b is formed of, for example, a thread of nylon or the like or a wire of stainless steel or the like.

The grip portion 20b in the third embodiment further includes a connection portion 26b. As illustrated in Fig. 7, the connection portion 26b is configured to be connectable to a hollow conduit 400 that is introduced from the outside through the scleral wound such as a third sclera/pars plana incision port 330. When the connection portion 26b is connected to the hollow conduit 400, a flow passage 22b of the grip portion 20b and the inside of the hollow conduit 400 communicate with each other. Therefore, the flow passage 22b formed inside the grip portion 20b of the third embodiment communicates with the first through-hole 12 of the loading portion 10 and communicates with a through-hole (not illustrated) formed inside the hollow conduit 400. In the present embodiment, the connection portion 26b is formed next to the proximal end side of the protrusion 24 at the same circumferential position as the protrusion 24, but may be formed next to the distal end side of the protrusion 24, or may be formed at a different circumferential position from the protrusion 24. One end of the hollow conduit 400 is connected to the connection portion 26b, and the other end of the hollow conduit 400 is exposed to the outside of the scleral wound and connected to an automatic or manual syringe, pump, or the like (not illustrated). The hollow conduit 400 may be formed by, for example, a flexible hollow tube, a rigid hollow needle, or the like.

As illustrated in Fig. 7, the cell transplantation instrument 100b inserted into the vitreous cavity 210 through the first sclera/pars plana incision port 310 is guided by the protrusion 24 of the grip portion 20b being gripped with the forceps 300 inserted into the vitreous cavity 210 through the second sclera/pars plana incision port 320. Then, the hollow conduit 400 inserted into the vitreous cavity 210 through the third sclera/pars plana incision port 330 or the like and the connection portion 26b of the grip portion 20b are connected together. A liquid is injected through the through-hole formed inside the hollow conduit 400, the flow passage 22b formed in the grip portion 20b, and the first through-hole 12 formed in the loading portion 10, whereby the cells in the loading portion 10 are transplanted by being carried on the water flow of the liquid.

According to the cell transplantation instrument 100b of the third embodiment described above, the operability in subretinal cell transplantation can be improved similarly to the cell transplantation instruments 100 and 100a of the first and second embodiments. In addition, since the connection portion 26b that brings the flow passage 22b of the grip portion 20b and the through-hole inside the hollow conduit 400 into communication with each other is provided, it is possible to inject the liquid into the cell transplantation instrument 100b through the hollow conduit 400 and transplant the cells loaded in the loading portion 10. Moreover, since the thread or the wire connected to the proximal end of the grip portion 20b is further provided, it is possible to easily remove the cell transplantation instrument 100b from the eyeball 200 after cell transplantation or the like while reducing or preventing complication of the configuration of the cell transplantation instrument 100b.

### E. Other Embodiments

The configurations of the cell transplantation instruments 100, 100a, and 100b of the above embodiments are merely examples, and various modifications can be made. For example, the hollow tube 30, the hollow needle 30a, and the small-diameter member 30b may be omitted. In addition, for example, the maximum dimension of the grip portion 20 or 20b in the cross section perpendicular to the longitudinal direction may be larger than the maximum dimension of the loading portion 10 in the cross section perpendicular to the longitudinal direction. In addition, for example, the distal end 14 of the loading portion 10 is not limited to the obliquely-cut shape, and may have any shape. In addition, the protrusion 24 of the grip portion 20 or 20b may be formed in a direction different from the cut surface 16 of the distal end 14 of the loading portion 10 in the circumferential direction. In addition, the shape of the protrusion 24 formed on the grip portion 20 or 20b is not limited to the thin plate-like shape extending along the longitudinal direction, and may be any shape that can be gripped with the forceps 300. In addition, the grip portion 20 is not limited to the protrusion 24, and may have a shape to be gripped with the forceps. The shape to be gripped with the forceps in the grip portion 20 is not particularly limited, and examples thereof include a shape having a narrowed portion, a hole, or a polygonal cross section. In addition, when the loading portion 10 is placed with the cut surface 16 of the distal end 14 facing upward, the shape to be gripped with the forceps in the grip portion 20 may also be provided to face upward. The shape to be gripped with the forceps may also be formed in any direction depending on the ease of insertion, in a bevel-up state when the loading portion 10 is placed with the cut surface 16 of the distal end 14 facing upward, and in a bevel-down state when the loading portion 10 is placed with the cut surface 16 of the distal end 14 facing upward.

The present invention is not limited to the above-described embodiments, and can be implemented in various configurations without departing from the gist of the present invention. For example, the technical features in the embodiments and examples corresponding to the technical features in the respective modes described in the summary of invention can be appropriately replaced or combined in order to partly or wholly solve the above-described problem or achieve some or all of the above-described effects. In addition, if the technical features are not described as essential in the present specification, they can be deleted as appropriate.

### [Reference Signs List]

10 Loading portion; 12 First through-hole; 14 Distal end; 16 Cut surface; 20, 20b Grip portion; 22, 22b Flow passage; 24 Protrusion; 26b Connection portion; 30 Hollow tube; 30a Hollow needle; 30b Small-diameter member; 32 Second through-hole; 33 Third through-hole; 100, 100a, 100b Cell transplantation instrument; 200 Eyeball; 210 Vitreous cavity; 220 Incision site; 300 Forceps; 310 First sclera/pars plana incision port; 320 Second sclera/pars plana incision port; 330 Third sclera/pars plana incision port; 400 Hollow conduit; 500 Cell transplantation instrument

## Claims

1. A cell transplantation instrument to a subretinal space, the cell transplantation instrument comprising:
a loading portion having a tubular shape and including a first through-hole formed along a longitudinal direction, the first through-hole being loaded with transplanted cells; and
a grip portion continuous with a proximal end of the loading portion and having a shape to be gripped with forceps, wherein
a flow passage communicating with the first through-hole is formed inside the grip portion.

2. The cell transplantation instrument according to claim 1, wherein
the shape is formed by a protrusion.

3. The cell transplantation instrument according to claim 1 or 2, further comprising
a hollow tube continuous with a proximal end of the grip portion and including a second through-hole formed along a longitudinal direction of the hollow tube, wherein
the hollow tube has flexibility, and
the second through-hole communicates with the flow passage.

4. The cell transplantation instrument according to claim 1 or 2, further comprising
a hollow needle continuous with a proximal end of the grip portion and including a third through-hole formed along a longitudinal direction of the hollow needle, wherein
the hollow needle has rigidity, and
the third through-hole communicates with the flow passage.

5. The cell transplantation instrument according to claim 1, further comprising
a thread or a wire continuous with a proximal end of the grip portion.

6. The cell transplantation instrument according to claim 1 or 5, wherein
the grip portion further includes a connection portion, and
the connection portion is connected to a hollow conduit that is introduced from an outside through a scleral wound, to bring the flow passage of the grip portion and an inside of the hollow conduit into communication with each other.

7. The cell transplantation instrument according to claim 1 or 2, wherein
a maximum dimension of the grip portion in a cross section perpendicular to the longitudinal direction of the loading portion is substantially equal to or smaller than a maximum dimension of the loading portion in the cross section.

8. The cell transplantation instrument according to claim 1 or 2, wherein
a distal end of the loading portion has an obliquely-cut shape.

9. The cell transplantation instrument according to claim 8, wherein
when the loading portion is placed with a cut surface of the distal end facing upward,
the shape is also provided to face upward.
